# EUROPÄISCHE PATENTSCHRIFT

(11) **EP 0 536 671 B1**
(45) Veröffentlichungstag und Bekanntmachung des Hinweises auf die Patenterteilung: **06.03.1996**
(21) Anmeldenummer: 92116964.5
(22) Anmeldetag: 05.10.1992
(51) Int. Cl.: C07K 1/06, C07K 1/12, C07C 229/08, C07C 229/36, C07C 271/22

(54) **Carbonsäureester-Schutzgruppen, ein Verfahren zu ihrer Herstellung, ihre Kopplung an eine funktionelle Gruppe sowie ihre Verwendung**
Carboxylic acid ester protecting groups, process to prepare them, their coupling to functional groups and their use
Groupes de protection ester d'acides carboxyliques, procédé pour les synthétiser, leur couplage à un groupe fonctionnel et leur utilisation

(30) Priorität: 07.10.1991 DE 4133139; 25.04.1992 DE 4213706
(43) Veröffentlichungstag der Anmeldung: 14.04.1993
(73) Patentinhaber: HOECHST AKTIENGESELLSCHAFT, D-65926 Frankfurt am Main (DE)
(72) Erfinder: Kunz, Horst, Prof.Dr., W-6500 Mainz-Drais (DE); Braum, Günther, W-6200 Wiesbaden (DE); Braun, Peter, W-6400 Mainz (DE)

(56) Entgegenhaltungen:
- EP-A- 0 145 956
- EP-A- 0 527 427
- DE-A- 352 831
- ANGEWANDTE CHEMIE,INT.ED.ENGL Bd. 11, Nr. 10, 1971, Seiten 811 - 812 MUTTER M.
- ET AL. 'Mew Method of Polypeptide Synthesis'
- CHEM.BERICHTE Bd. 107, Nr. 4, 1974, Seiten 1344 - 1352 E.BAYER ET AL. 'Synthese des biologisch aktiven Undecapeptids Substanz P nach der Fl ssig-Phasen- Methode'
- LIEBIGS ANN.CHEM. Bd. 2, 1991, Seiten 165 - 170 P.BRAUN ET AL. 'Selektive enzymatische Schutzgruppenabspaltungen: Der n-Heptylester als Carboxylschutzgruppe in der Peptidsynthese'
- SYNLETT Februar 1990, Seiten 105 - 107 P.BRAUN ET AL. 'Selective Enzymatic Removal of Protecting Functions: Heptyl Esters as Carboxy Protecting Groups in Peptide Synthesis'

## Beschreibung

### Verfahren zur Peptidsynthese unter Verwendung enzymatisch abspaltbarer Carbonsäureester-Schutzgruppen

Die Erfindung betrifft ein Verfahren zur Peptidsynthese unter Verwendung von polaren, hydrophilen, C-terminalen Carbonsäureesterschutzgruppen, welche sich enzymatisch mittels Lipasen spezifisch abspalten lassen.

Als Schutzgruppe wird ein organischer Rest bezeichnet, mit dem funktionelle Gruppen eines mehrere aktive Zentren enthaltenden Moleküls vorübergehend gegen den Angriff von Reagenzien geschützt werden können, so daß Reaktionen nur an den gewünschten (ungeschützten) Stellen stattfinden. Nach Beendigung der Reaktion sollte sich die Schutzgruppe selektiv abspalten lassen (Römpps Chemielexikon, Franckh Fachlexikonverlag, Stuttgart, Deutschland, Bd. 5, S. 3744-3745).

Als geeigneter Schutz von Peptiden am C-terminalen Ende wird von Mutter et al. (Angew. Chem. int. Edit. 10, 811-812 (1971); Chem. Ber. 1078, 1344-1352 (1974)) beispielsweise die Veresterung der C-terminalen Carboxylgruppen mit Polyethylenglycolresten vorgeschlagen (PEG-Ester).

Aus der DE 35 28 631 A1 sind ferner Diethylenglycolmonomethylester (Dem) von Aminosäurederivaten bekannt.

Die EP 0 527 427 A2 und die EP 0 145 956 A1 offenbaren ferner Aminosäurederivate, welche mit 2-(Morpholino)-ethanol verestert sind.

Lipasen sind Esterhydrolasen, die bekanntermaßen Ester von Fettsäuren mit Glycerin oder anderen Alkoholen und solche von Carbonsäuren mit Fettalkoholen hydrolysieren [G.M. Whitesides, C.H. Wong, Angew. Chem. 97 (1985), 617]. Lipasen werden u.a. zur Schutzgruppenablösung von Peptiden erfolgreich eingesetzt. So z.B. zur enzymatischen Spaltung von Aminosäure- und Peptid-n-Heptyl-(Hep)-estern, die aus unpolaren Alkoholen hergestellt werden. Diese Ester sind das natürliche Substrat der Lipasen. (P. Braun, H. Waldmann, W. Vogt, H. Kunz, Syntlett 1990, 105). Es zeigt sich dabei jedoch eine starke Strukturabhängigkeit der enzymatischen Spaltbarkeit. Ausgesprochen hydrophobe Peptidester, wie z.B. Boc-Val-Phe-OHep, werden von den bisher bekannten Lipasen grundsätzlich nicht angegriffen (P. Braun, W. Vogt und H. Kunz, Liebigs Ann. Chem. 1991, 165-170).

In dem japanischen Patent 119260 ist beschrieben, daß Carbonsäurester hydrophiler Alkohole von Amidoacylasen erkannt werden. Das durch die enzymatische Spaltung entstandene Carbonsäurederivat wird jedoch nicht als Schutzgruppe eingesetzt, sondern mit einem weiteren Reaktanten zum Amid umgesetzt.

In C. A. 95 (19) 1691902g ist die oben beschriebene enzymatische Spaltung ebenfalls beschrieben, jedoch wird anstelle des freien Enzyms Amidoacylase der gesamte Mikroorganismus Bacillus circulans M-1123-5 eingesetzt, der auch Amidoacylase-Aktivität besitzt.

Es wurde nun überraschend gefunden, daß sich insbesondere Aminosäuren, Peptide und Glycopeptide vorteilhafterweise C-terminal als Carbonsäureester polarer hydrophiler Alkohole schützen lassen, da sie sich selektiv einführen lassen und spezifisch durch Lipasen abspaltbar sind.

Die Erfindung betrifft ein Verfahren zur Peptidsynthese, das sich dadurch auszeichnet, daß eine Aminosäure, ein Peptid oder ein Glycopeptid, deren N-terminale Aminogruppe geschützt oder ungeschützt ist und welche weitere funktionelle Gruppen in geschützter oder ungeschützter Form tragen können, zu einer carboxyterminal geschützten Verbindung der allgemeinen Formel I 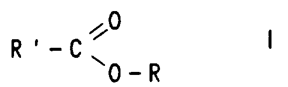 verestert wird,
worin bedeuten
R ein unverzweigter oder verzweigter organischer Rest, der als polare Glieder zwischen aliphatischen oder araliphatischen Kohlenwasserstoffbrücken Ethersauerstoffe, Aminstickstoffgruppierungen oder ein Gemisch aus Ether- und Amingruppierungen enthält, die in eine Ringstrukturen eingebunden sein können, wobei die Gesamtlänge nicht größer als 20 Glieder ist, im Falle von Polyethylenglykol [(CH₂-CH₂-O)ₙ], n die Anzahl der Glieder angibt und als beliebig große ganze Zahl definiert wird
und 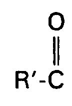 einen Aminosäurerest, einen Peptidrest oder einen Glycopeptidrest, die N-terminale Aminogruppe der Verbindungen der Formel I notwendigenfalls entschützt und die Verbindung der Formel 1 mit N-geschützten Aminosäuren zu höheren Peptideinheiten verknüpft, wonach die carboxyterminale Schutzgruppe unter enzymatischer Katalyse mittels einer in Wasser oder in wasserhaltigen Lösungen gelösten Lipase abgespalten wird.

Vorzugsweise haben die Kohlenwasserstoffbrücken zwischen den polaren Gliedern im Rest R Ethylenglieder der Struktur -CH₂-CH₂- und die polaren Glieder zwischen den Ethylen-Brücken im Rest R sind Sauerstoff.

Wahlweise sind die polaren Glieder zwischen den Ethylen-Brücken im Rest R Stickstoffunktionen der Struktur N-R mit R =H oder R =Niederalkyl (C₁-C₆), wobei diese mit den Ethylen-Brücken auch zu Cyclen verknüpft sein können.

Die polaren Glieder im Rest R können sowohl Sauerstoff als auch Aminstickstoffunktionen sein.

Die Gruppierung R'-CO in der Verbindung der Formel 1 ist vorzugsweise ein Peptid bestehend aus 1 bis 100 Aminosäuren, vorzugsweise aus 1 bis 50 und ganz besonders bevorzugt aus 1 bis 25 Aminosäuren, oder ein Glycopeptid.

In einer bevorzugten Ausführungsform der vorliegenden Erfindung wird eine Verbindung der Formel I eingesetzt, in welcher R ein Diethylenglycolmonomethyleter-Rest (DEM) ist, welcher sich durch Umsetzung der Aminosäure, des Peptids oder des Glycopeptids mit dem entsprechenden freien Alkohol unter den Bedingungen der azeotropen Veresterung herstellen läßt.

In einer weiteren bevorzugten Ausführungsform ist die Verbindung der Formel I ein 2-(Morpholino)-ethylester, der sich durch Umsetzung des 2-Halogenethylesters der Aminosäure, des Peptids oder des Glycopeptids mit Morpholin herstellen läßt.

Eine ferner bevorzugte Ausführungsform der vorliegenden Erfindung ist der Einsatz von Polyethylenglycol-(PEG)-Estern einer Aminosäure, eines Peptids oder eines Glycopeptids.

Bei dem Verfahren gemäß der vorliegenden Erfindung erfolgt die enzymatische Hydrolyse der Verbindung der Formel I durch Katalyse mit einer in Wasser bzw. in wasserhaltigen Lösungen gelösten Lipase in Gegenwart von komplexierenden lonen, vorzugsweise Alkali-lonen.

Im folgenden wird die Erfindung detailliert beschrieben.

Als araliphatisch werden organische Kohlenwasserstoffverbindungen definiert, die sowohl aromatische wie aliphatische Reste enthalten.

Die Verbindung der Formel I wird aus polaren, hydrophilen Alkoholen und einer Aminosäure, einem Peptid oder einem Glycopeptid nach dem Fachmann bekannten Verfahren hergestellt.

Bei der Verbindung der Formel I handelt es sich insbesondere Ester mit Diethylenglykolmonomethylether (Dem) der Struktur 1, mit 2-(Morpholino)-ethylester (MoEt) der Struktur 2 und mit PEG (Polyethylenglycol) der Struktur 3 von Aminosäuren, Peptiden oder Glycopeptiden.

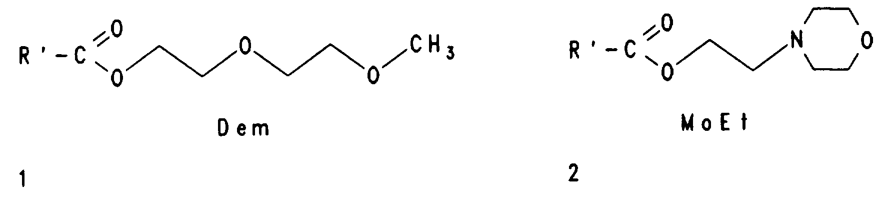

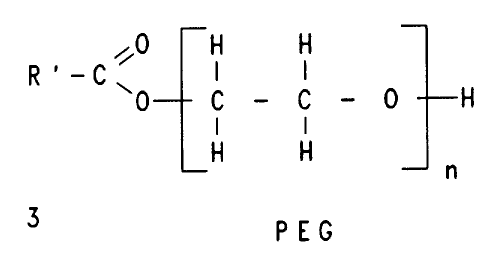

Die Moleküle besitzen abgesehen von der geschützten funktionellen Gruppe mindestens noch eine weitere funktionelle Gruppe, wo sinnvollerweise die Reaktion stattfindet. Sind zwei oder mehr funktionelle Gruppen innerhalb eines Moleküls vorhanden, können außer der oben erwähnten Schutzgruppe weitere eingeführt werden. Hierfür können die gleichen oder verschiedene Schutzgruppen verwendet werden.

Die Größe der Peptide beträgt vorzugsweise 1 bis 100 Aminosäuren, insbesondere jedoch 1 bis 50 Aminosäuren und ganz vorzugsweise 1 bis 25 Aminosäuren.

Besondere Eigenschaft der Verbindungen der Formel 1 ist, daß der alkoholische Teil R polare, hydrophile Eigenschaften hat, wie sie z.B. durch Oligo- und Polyether-Strukturen vom Ethylenglykol-Ester-Typ oder durch Amin- und Ethergruppen in der Kette bewirkt werden. Die Verbindungen der Formel 1 sind dann noch immer ausreichend in Wasser bzw. in wasserhaltigen Lösemitteln löslich, wenn der Acyl-Rest R' hydrophoben Charakter hat. Das trifft insbesondere auf N-terminal und in den Seitenketten geschützte Aminosäure-und Peptidester zu, deren Carboxylgruppen in der vorbezeichneten Esterform geschützt sind.

Die Herstellung der Verbindungen der Formel I erfolgt nach dem Fachmann bekannten Verfahren. Zum Beispiel setzt man Aminosäuren mit Diethlenglykolmonomethylether unter den Bedingungen der azeotropen Veresterung um. Die Umsetzung kann außerdem nach dem Verfahren von Kunz und Buchholz (Chem. Ber. 112 (1979, 2145) bzw. analog dem Verfahren von Buchholz und Kunz (Liebigs. Ann. Chem. 1983, 1859) erfolgen. Diese beiden Verfahren werden vorzugsweise bei MoET eingesetzt. Außerdem ist die Kopplung nach dem von Mutter und Bayer (M. Mutter und E. Bayer in "The Peptides" Academic Press, New York 1979, Kapitel 2, S. 286-329) entwickelten Verfahren möglich, vorzugsweise bei Polyethylenglykol.

Wie oben schon erwähnt, spalten Lipasen die Schutzgruppen spezifisch ab. Hierfür können alle handelsüblich erhältlichen Lipasen verwendet werden.

Vor Beginn der enzymatischen Spaltung muß getestet werden, ob die Lipase durch Proteasen kontaminiert ist. Proteasen müssen nach dem Fachmann bekannten Methoden durch Phenylmethylsulfonylfluorid (PMSF) blockiert werden.

Die enzymatische Abspaltung der polaren, hydrophilen Carboxylschutzgruppen durch Lipasen erfolgt nach dem Fachmann bekannten Methoden in wasserhaltigen Medien, vorzugsweise 90 % Wasser und 10 % Aceton (v/v), bei pH 7,0. Die Reaktionszeit beträgt 12 bis 48 Stunden bei 37°C.

### Beispiel 1:

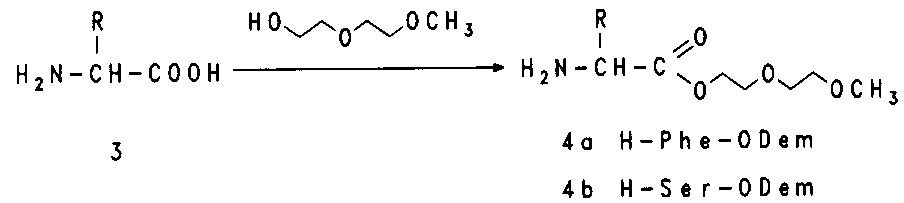

Die 2-(Morpholino)ethylester werden günstigerweise aus den 2-Brom-ethylestern der Aminosäure bzw. Peptide und Glycopeptide, hergestellt nach der Vorschrift von H. Kunz, M. Buchholz, Chem. Ber. 112 (1979), 2145 bzw. analog zu dem Verfahren von M. Buchholz, H. Kunz, Liebigs. Ann. Chem. 1983, 1859, durch Umsetzung mit Morpholin erhalten (siehe Beispiele 2 und 3).

### Beispiel 2a:

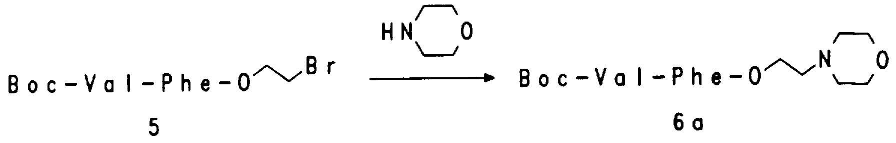

### Beispiel 3:

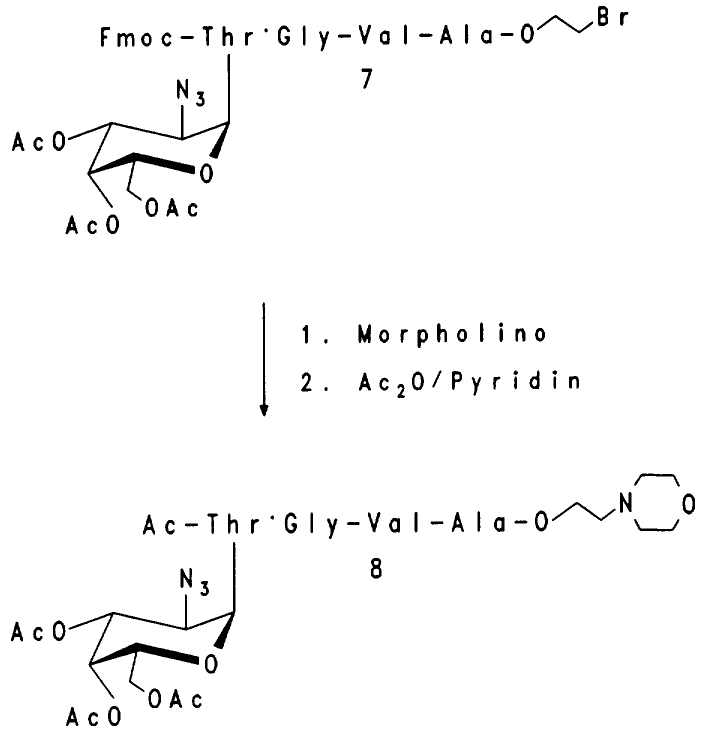

Die gewonenen Aminosäureester werden mit N-geschützten Aminosäuren bzw. Peptiden zu höheren Peptideinheiten verknüpft. (Beispiel 4)

### Beispiel 4a:

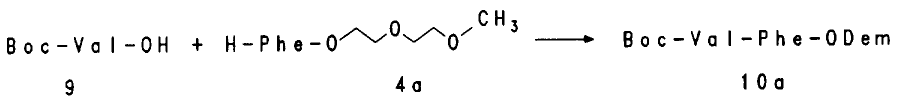

Von den so gewonnenen C-terminal erfindungsgemäß geschützten Peptiden und Glycopeptiden werden die polaren, hydrophilen Carboxylschutzgruppen durch Lipasen (s. Beispiel 5a - h) in wasserhaltigen Medien (in der Regel Wasser und 10 % v/v Aceton) bei ph 7 in 12 Stunden bis 48 Stunden (bei komplexen Glycopeptiden wie 8) abgespalten. Da die Bedingungen neutral und außerordentlich mild sind (37°C), bleiben alle anderen Schutzgruppen an den teilweise sehr komplexen Peptiden und Glycopeptiden erhalten. Das gilt für die basenlabile Fmoc-, die reduktiv entfernbaren Z und Trichlorethoxycarbonyl (Teoc)-Gruppen ebenso wie für säurelabile Gruppen, z.B. die Boc-Gruppe.

### Beispiel 5a - h:

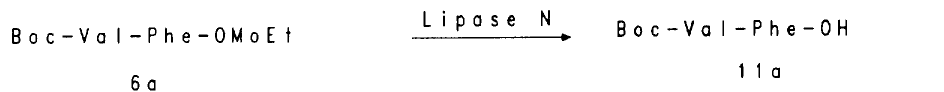

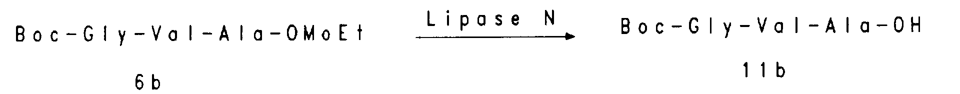

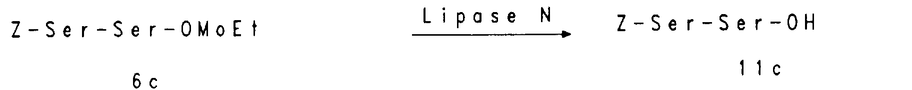

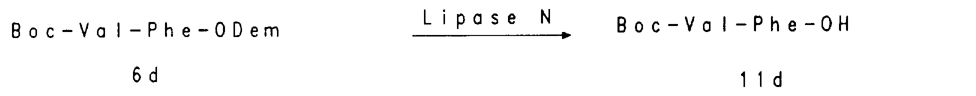

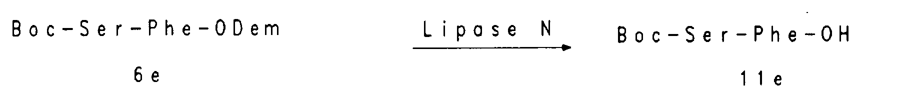

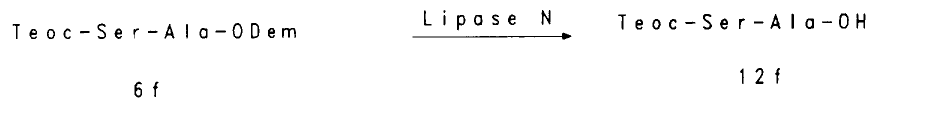

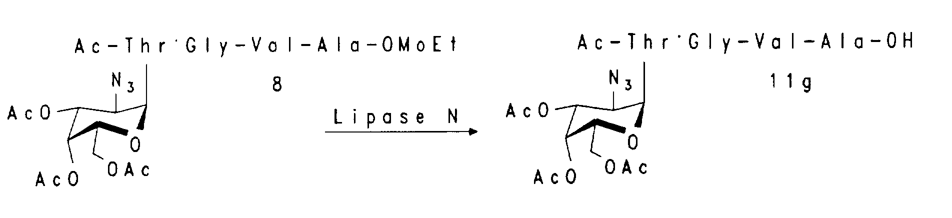

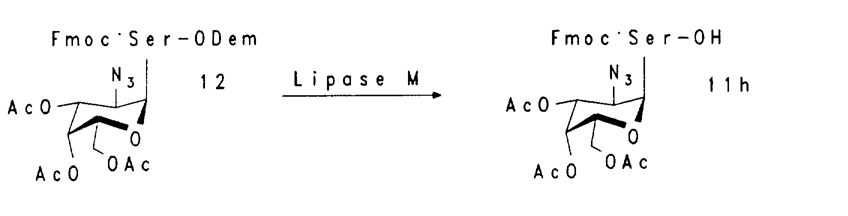

Die in Lipase-Präparationen häufig enthaltenen Proteasen müssen sorgfältig durch Zugabe von Phenylmethylsulfonylfluorid (PMSF) blockiert werden. Die Ergebnisse der enzymatischen Schutzgruppenabspaltung zeigen, daß die erfindungsgemäß verwendeten Ester, hier mit Diethylenglykol- und 2-(Morpholino)ethyl-Struktur, trotz ihres polaren, hydrophilen Charakters von den Lipase als Substrate akzeptiert und hydrolysiert werden. Sogar jene Peptidsequenzen, wie die hydrophoben Verbindungen 6 und 10a, deren Heptylester nicht angegriffen wurden, werden ohre Schwierigkeiten hydrolysiert. Daraus folgt, daß die durch Lipasen spaltbaren polaren Ester nicht nur an fettähnlichen sondern auch an polaren Carboxykomponenten enzymatisch entfernt werden können.

### Beispiele 6 und 6a: Synthese von Estern der Struktur 1 Allgemeine Vorschrift zur Herstellung von Aminosäureestern mit Diethylenglykolmonomethylether 4

Man erhitzt ein Gemisch aus 0,15 mol Aminosäure 3, 0,8 mol Diethylenglykolmonomethylether, 0,18 mol p-Toluolsulfonsäure-Hydrat und 150 ml Benzol unter Wasserabscheidung und Rückfluß, bis sich kein Wasser mehr abscheidet (24 Stunden). Nach Beendigung der Reaktion werden das Benzol im Wasserstrahlvakuum und danach der überschüssige Diethylenglykolmonomethylether im Hochvakuum abdestilliert. Zur Reinigung wird der Rückstand mehrmals mit Ether digeriert und anschließend im Hochvakuum getrocknet. Die Verbindungen 4a und 4b werden zu den geschützen Dipeptiden 10a und 10b umgesetzt und als solche analytisch charakterisiert.
a) L-Phenylalanin-diethylenglykol(monomethylether)-ester-hydro-p-toluolsulfonat 4a
b) L-Serin-diethylenglykol(monomethylether)-ester-hydro-p-toluolsulfonat 4b

### Beispiele 7a - c: Synthese von Estern der Struktur 2

### Allgemeine Vorschrift zur Herstellung von 2-Morpholino-ethylestern aus Bromund Jodethylestern

Eine Lösung von 5 mmol 2-Halogenethylester in 15 ml Morpholin wird 1 bis 4 Stunden bei 25°C gerührt. Anschließend destilliert man das Morpholin im Vakuum ab, der verbleibende Rückstand wird in Ether aufgenommen und mit eiskalter 0,5 N Salzsäure ausgeschüttelt. Die vereinigten wäßrigen Phasen werden mit Natriumhydrogencarbonat auf pH 9 eingestellt und mit Methylenchlorid extrahiert. Zur Reinigung wird der erfindungsgemäß geschützte Morpholino-ethylester nach Trocknen über Magnesiumsulfat und Abdestillieren des Lösungsmittels im Vakuum mit wenig Ether zur Kristallisation gebracht.
a) N-tert-Butyloxycarbonyl-L-valyl-L-phenylalanin-2-morpholino-ethylester 6a
   Ausbeute: 95 %, Schmp.: 83 bis 84°C, [α]_{D} ²² = 15,6 (c = 1; CHCl₃).
b) N-tert-Butyloxycarbonyl-glycyl-L-valyl-L-alanin-2-morpholino-ethylester 6b
   Ausbeute: 64 %, Schmp.: 115 bis 116°C, [α]_{D} ²² = 0,9 (c = 1,1; CHCl₃).

### Beispiel 8: Synthese eines Glycopeptidesters der Struktur 2

Die Herstellung von N-Acetyl-3-O-(3,4,6-tri-O-acetyl-2-azido-2-desoxy-α-D-galactopyranosyl)-L-threonyl-glycyl-L-valyl-L-alanin-2-morpholino-ethylester8 erfolgt nach einer Variante der allgemeinen Vorschrift von Beispiel 2:

Man rührt 1,5 g (1,5 mmol) N-(9-Fluorenylmethoxycarbonyl)-3-O-(3,4,6-tri-O-acetyl-2-azido-2-desoxy-β-D-galactopyranosyl)-L-threonyl-glycyl-L-valyl-L-alanin-2-brom-ethylester 7 in 5 ml frisch destilliertem Morpholin 30 Minuten bei Raumtemperatur. Die Reaktionsmischung wird im Vakuum bei maximal 40°C Badetemperatur eingeengt und mehrfach mit Toluol und Ether codestilliert, bis das Morpholin vollständig entfernt ist. Der Rückstand wird mit 10 ml einer gekühlten Mischung von Acetanhydrid/Pyridin (2 : 1) versetzt und 2 Stunden bei 20°C gerührt. Anschließend wird die Reaktionsmischung im Vakuum eingeengt und mehrfach mit Toluol codestilliert. Den so erhaltenen Rückstand löst man in 50 ml Methylenchlorid und extrahiert mehrfach mit eiskalter 0,5 N Salzsäure. Die gesammelten wäßrigen Phasen werden mit Natriumhydrogencarbonat auf pH 8,5 eingestellt und mit Methylenchlorid ausgeschüttelt. Die vereinigten organischen Phasen werden mit 20 ml gesättigter Natriumchlorid-Lösung gewaschen, über Magnesiumsulfat getrocknet und im Vakuum eingeengt. Der Rückstand wird an 80 g Kieselgel 60 (Fa. Merck, Darmstadt, Deutschland) durch Flash-Chromatographie (Laufmittel CH₂Cl₂/EtOH, 10 : 1) gereinigt.
Ausbeute: 0,53 g (0,65 mmol); 43 %, [α]_{D} ²² = 54,4 (c = 1; CHCl₃).

### Beispiele 9a - c: Synthese eines Peptidesters der Struktur 1

### Ngeschützter Dipeptid-Dem-ester 10 mit Hilfe von 1-Ethoxycarbonyl-2-ethoxy-1,2-dihydro-chinolin (EEDQ,O)

Zu einer Lösung von 0,01 mol Aminosäure-Dem-ester-hydro-p-toluolsulfonat 4 und 0,01 mol Di-isopropyl-ethyl-amin in 20 ml Dichlormethan gibt man eine Lösung von 0,01 mol N-geschützte Aminosäure 9 in 20 ml Dichlormethan und 0,012 mol EEDQ und läßt 12 Stunden bei Raumtemperatur rühren. Anschließend wird die Reaktionsmischung je dreimal mit 0,5 N HCI, 0,5 N NaHCO₃ und Wasser extrahiert, über Magnesiumsulfat getrocknet und im Vakuum eingeengt. Das Rohprodukt wird durch Flash-Chromatographie (Petrolether/Essigester) gereinigt.
a) N-tert-Butyloxycarbonyl-L-valyl-L-phenylalanin-Dem-ester (10a) Ausbeute: 71 %, wachsartige Substanz, [α]_{D} ²² = 23,3 (c = 0,9; CHCl₃).
b) N-tert-Butyloxycarbonyl-L-seryl-L-phenylalanin-Dem-ester (10b) Ausbeute: 64 %, wachsartige Substanz, [α]_{D} ²² = -6,6 (c = 1,0; CHCl₃).
c) N-Trichlorethyloxycarbonyl-L-alanyl-L-serin-Dem-ester (10c) Ausbeute: 74 %, Öl, [α]_{D} ²² = -3,4 (c = 1,1; CHCl₃).

### Beispiel 10a - h: Enzymatische Spaltung der Ester der Struktur 1 bzw. 2

### Allgemeine Vorschrift zur enzymatischen Abspaltung der geschützten Ester

1. Inhibierung der Proteaseaktivität der Enzympräparation:
   200 mg Lipase N (Fa. Amano) werden in 1 ml 0,2 M Phosphatpuffer (pH 7) gelöst, mit 3,5 mg Phenylmethylsulfonylfluorid (PMSF) versetzt und je 1 Stunde bei 0°C und 37°C gerührt.
2. C-terminale Deblockierung:
   Die Enzymlösung wird mit Phosphatpuffer auf 25 ml aufgefüllt und mit einer Lösung von 200 mg erfindungsgemäß geschütztem Ester in höchstens 2,5 ml Aceton 16 Stunden bei 37°C gerührt. Danach wird die mit Natriumhydrogencarbonat auf pH 8,5 eingestellte Reaktionslösung mit 10 ml Wasser verdünnt und mit Ether extrahiert. Die durch Kaliumhydrogensulfat auf pH 4 eingestellt Wasserphase wird mit Essigester ausgeschüttelt. Die vereinigten Essigesterphasen werden über Magnesiumsulfat getrocknet und im Vakuum eingeengt. Das carboxy-deblockierte Produkt kann, sofern nötig, durch Flash-Chromatographie unter Verwendung einer Essigester/Ethanol-Mischung gereinigt werden. So werden aus den entsprechenden 2-Morpholinoethylestern folgende Verbindungen erhalten:

a) N-tert-Butyloxycarbonyl-L-valyl-L-phenylalanin 11a
   Ausbeute: 91 %, amorph, [α]_{D} ²² = 14,2 (c = 1,0; CHCl₃).
b) N-tert-Butyloxycarbonyl-glycyl-L-valyl-L-alanin 11 b
   Ausbeute: 78 %, Schmp.: 179°C, [α]_{D} ²² = -31,9 (c = 1,0; CH₃OH).
c) N-Benzyloxycarbonyl-L-seryl-serin 11c
   Ausbeute: 78 %, amorph, [α]_{D} ²² = 12,1 (c = 0,6; CH₃OH).

Aus den entsprechenden Diethylenglycolmonomethylestern werden die Verbindungen 11d - f gewonnen:
Die durch enzymatische Hydrolyse der Dem-Ester erhaltenen Dipeptidsäuren 11d - f werden als Dicyclohexylamin(DCHA)-Salze charakterisiert.
d) N-tert-Butyloxycarbonyl-L-valyl-L-phenylalanin * DCHA 11d
   Ausbeute: 97 %, amorph, [α]_{D22} = -14,3 (c = 1,0; CH₃OH).
e) N-tert-Butyloxycarbonyl-seryl-L-phenylalanin * DCHA 11e
   Ausbeute: 94 %, Schmp.: 184°C, [α]_{D} ²² = 15,4 (c = 1,0; CH₃OH).
f) N-Trichlorethyloxycarbonyl-L-alanyl-L-serin * DCHA 11f
   Ausbeute: 92 %, Schmp.: 172 bis 175°C, [α]_{D} ²² = -1,0 (c = 0,65; CH₃OH).

Zur Spaltung der erfindungsgemäßen Estergruppen an Glycopeptiden wird aus 400 mg Lipase eine Enzym-Lösung bereitet, die nach maximal 48 Stunden Reaktionszeit mit Natriumchlorid gesättigt und mit Essigester extrahiert wird. Die über Magnesiumsulfat getrocknete Essigesterphase wird im Vakuum eingeengt und der so erhaltene Rückstand an Kieselgel chromatorgraphisch gereinigt.

Aus dem entsprechenden 2-Morpholino-ethylester 8 erhält man:
g) N-Acetyl-3-O-(3,4,6-tri-O-acetyl-2-azido-2-desoxy-α-D-galactopyranosyl)-L-threonyl-glycyl-L-valyl-L-alanin 11g
   Ausbeute: 59 %; amorpher Feststoff, [α]_{D} ²² = 56,2 (c = 0,54; MeOH).

Aus dem entsprechenden Diethylenglycolmonomethyl-ester 12 erhält man das Glycopeptid 11h, wobei zur enzymatischen Hydrolyse unter den oben beschriebenen Versuchsbedingungen Lipase M (Fa. Amano) verwendet wird.
h) N-(9-Fluorenylmethoxycarbonyl)-3-0-(3,4, 6-tri-O-acetyl-2-azido-2-desoxy-α-D-galactopyranosyl)-L-serin 11h Ausbeute: 64 %, Schaum, [α]_{D} ²² = 92,2 (c = 1,1; CH₃OH).

### Beispiel 11: Vorschrift zur Synthese von Peptiden an einer Polyethylenglycolmatrix

a) Vorschrift zur Veresterung der C-terminalen Aminosäure mit Polyethylenglycol:
   Die Kopplung wird nach einer von M. Mutter und E. Bayer beschriebenen Methode vorgenommen. In 10 ml Methylenchlorid werden 8 mmol N-(9-Fluorenylmethoxycarbonyl)-L-aminosäure mit 820 mg (mmol) Dicyclohexylcarbodiimid 30 Minuten bei Raumtemperatur gerührt. Das gebildete Anhydrid wird durch Filtration vom ausgefallenen Harnstoff getrennt und im vierfachen Überschuß zu dem im Hochvakuum getrockneten Polyethylenglycol (MW 6000 gegeben. Die Reaktionslösung wird mit 1 ml Pyridin versetzt, eingeengt und bei Raumtemperatur gerührt. Nach 6 Stunden wird im Vakuum bis zur Trockne eingeengt, und in 20 ml Methylenchlorid gelöst. Der Polyethylenglycolester wird aus dieser Lösung unter Rühren bei -10°C durch Zugabe von ca. 150 ml absolutem Ether auskristallisiert. Nach beendeter Etherzugabe rührt man noch 10 Minuten bei -10°C, saugt scharf über eine Fritte ab, wäscht mehrmals mit Ether nach und trocknet über P₂O₅.

Wenn, insbesondere bei trifunktionellen Aminosäuren, eine nicht vollständige Veresterung befürchtet werden muß, werden die unumgesetzten OH-Gruppen mit Acetanhydrid/Pyridin blockiert. Die Reinigung erfolgt wie oben beschrieben durch Kristallisation.

Allgemeine Vorschrift zur N-terminalen Abspaltung der Fmoc-Gruppe und Verlängerung nach dem Carbodiimid Verfahren:

Der Polyethylenglycolester wird in der 10-fachen Menge Morpholin gelöst und eine Stunde bei Raumtemperatur gerührt. Die deblockierte Verbindung wird wie oben beschrieben durch Kristallisation gereinigt und sofort weiter verlängert. Dazu wird der N-terminal deblockierte Polyethylenglycolester mit 4 Äquivalenten N-(9-Fluorenylmethoxycarbonyl)-L-aminosäure, 6 Äquivalenten DCC und 8 Äquivaltenten HOBt in Methylenchlorid/DMF umgesetzt. Nach 4 bis 6stündigem Rühren bei 25°C wird der ausgefallene Harnstoff abgetrennt und wie üblich aufgearbeitet.

Auf diese Weise wurden folgende Peptide synthetisiert:
N-(9-Fluorenylmethoxycarbonyl-glycyl-L-valin-PEG 6000-ester
N-(9-Fluorenylmethoxycarbonyl-L-valyl-L-alanin-PEG 6000-ester
N-(9-Fluorenylmethoxycarbonyl-L-prolyl-L-prolyl-L-alanin-PEG 6000-ester

Diese Ester lassen sich ebenfalls enzymatisch spalten. Für die Berechnung der Ausbeuten wird angenommen, daß alle Kupplungsschritte quantitativ erfolgt sind. Durch ¹H-200MHz-NMR-Spektroskopie konnte jeweils in eindeutiger Weise die erfolgreiche Abspaltung des Polyethylenglycols und die Richtigkeit der Struktur der Peptide nachgewiesen werden.
N-(9-Fluorenylmethoxycarbonyl-glycyl-L-valin
   Ausbeute: 90 %, Schmp.: 69 bis 71°C, [α]_{D} ²² = -0,5 (c = 1,0; CH₃OH).
N-(9-Fluorenylmethoxycarbonyl-L-valyl-L-alanin
   Ausbeute: 66 %, Schmp.: 122 bis 123°C, [α]_{D} ²² = -44,3 (c = 1,1; CH₃OH).
N-(9-Fluorenylmethoxycarbonyl-L-prolyl-L-prolyl-L-alanin
   Ausbeute: 50 %, Schmp.: amorph, [α]_{D} ²² = -72,5 (c = 1,0; CH₃OH).

FAB-MS (Negativ-lonen-Detektion): m/z = 505 (M-1)
Matrix: Glycerin

## Patentansprüche

1. Verfahren zur Peptidsynthese, dadurch gekennzeichnet, daß eine Aminosäure, ein Peptid oder ein Glycopeptid, deren N-terminale Aminogruppe geschützt oder ungeschützt ist und welche weitere funktionelle Gruppen in geschützter oder ungeschützter Form tragen können, zu einer carboxyterminal geschützten Verbindung der allgemeinen Formel I 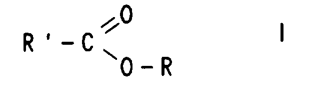 verestert wird,
worin bedeuten
R ein unverzweigter oder verzweigter organischer Rest ist, der als polare Glieder zwischen aliphatischen oder araliphatischen
Kohlenwasserstoffbrücken Ethersauerstoffe, Aminstickstoffgruppierungen oder ein Gemisch aus Ether- und Amingruppierungen enthält, die in eine Ringstrukturen eingebunden sein können, wobei die Gesamtlänge nicht größer als 20 Glieder ist, im Falle von Polyethylenglykol [(CH₂-CH₂-O)ₙ], n die Anzahl der Glieder angibt und als beliebig große ganze Zahl definiert wird und 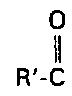
einen Aminosäurerest, einen Peptidrest oder einen Glycopeptidrest, die N-terminale Aminogruppe der Verbindungen der Formel I notwendigenfalls entschützt und die Verbindung der Formel I mit N-geschützten Aminosäuren zu höheren Peptideinheiten verknüpft, wonach die carboxyterminale Schutzgruppe unter enzymatischer Katalyse mittels einer in Wasser oder in wasserhaltigen Lösungen gelösten Lipase abgespalten wird.

2. Verfahren nach Anspruch 1, dadurch gekennzeichnet, daß die Kohlenwasserstoffbrücken zwischen den polaren Gliedern im Rest R Ethylenglieder der Struktur -CH₂-CH₂- sind.

3. Verfahren nach Anspruch 1 oder 2, dadurch gekennzeichnet, daß die polaren Glieder zwischen den Ethylen-Brücken im Rest R Sauerstoff sind.

4. Verfahren nach Anspruch 1 oder 2, dadurch gekennzeichnet, daß die polaren Glieder zwischen den Ethylen-Brücken im Rest R Stickstoffunktionen der Struktur N-R mit R = H oder R = Niederalkyl (C₁-C₆) sind, wobei diese mit den Ethylen-Brücken auch zu Cyclen verknüpft sein können.

5. Verfahren nach einem der Ansprüche 1 bis 4, dadurch gekennzeichnet, daß die polaren Glieder im Rest R sowohl Sauerstoff als auch Aminstickstoffunktionen sind.

6. Verfahren nach einem der Ansprüche 1 bis 5, dadurch gekennzeichnet, daß die Gruppierung R'-CO ein Peptidrest ist, welcher aus 1 bis 100 Aminosäuren besteht.

7. Verfahren nach Anspruch 6, dadurch gekennzeichnet, daß der Peptidrest aus 1 bis 50 Aminosäuren besteht.

8. Verfahren nach Anspruch 7, dadurch gekennzeichnet, daß der Peptidrest aus 1 bis 25 Aminosäuren besteht.

9. Verfahren nach einem der Ansprüche 1 bis 8, dadurch gekennzeichnet, daß die Gruppierung R'-CO ein Glycopeptidrest ist.

10. Verfahren nach einem der Ansprüche 1 bis 9, dadurch gekennzeichnet, daß R in Formel ein Diethylenglycolmonomethylether-Rest (Dem) ist.

11. Verfahren nach Anspruch 10, dadurch gekennzeichnet, daß eine Verbindung der Formel R'-COOH, in denen R' die in Anspruch 1 genannten Bedeutungen hat, mit einem Alkohol der Formel R-OH, in denen R die in Anspruch 10 genannte Bedeutung hat, unter den Bedingungen der azeotropen Veresterung zu einer Verbindung der Formel 1 gemäß Anspruch 10 umgesetzt wird.

12. Verfahren nach einem der Ansprüche 1 bis 9, dadurch gekennzeichnet, daß die Verbindung der Formel I ein 2-(Morpholino)-ethylester ist.

13. Verfahren nach einem der Ansprüche 1 bis 9, dadurch gekennzeichnet, daß die Verbindung der Formel I ein Polyethylenglykol-(PEG)-Ester ist.

14. Verfahren nach Anspruch 12, dadurch gekennzeichnet, daß ein Halogenalkylester der Formel R'-COO-CH₂-CH₂-X, wobei X Cl, Br oder I bedeutet, mit Morpholin zu dem 2-(Morpholino)-ethylester umgesetzt wird.

15. Verfahren nach einem der Ansprüche 1 bis 14, dadurch gekennzeichnet, daß die enzymatische Hydrolyse durch Katalyse mit einer in Wasser bzw. in wasserhaltigen Lösungen gelösten Lipase in Gegenwart von komplexierenden lonen, vorzugsweise Alkali-lonen erfolgt.

## Claims

1. A process for peptide synthesis which comprises esterifying an amino acid, a peptide or a glycopeptide, whose N-terminal amino group is protected or unprotected and which can have attached to it further functional groups in protected or unprotected form, to give a compound of the formula I 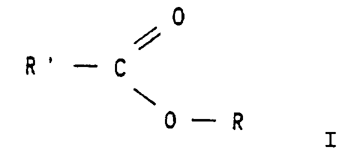 which is protected at the carboxyl end and in which R is an unbranched or branched organic radical which contains ether oxygens, amine nitrogen groups or a mixture of ether and amine groups as polar members between aliphatic or araliphatic hydrocarbon bridges which can be incorporated in a ring structure whose overall length does not exceed 20 members, in the case of polyethylene glycol [(CH₂-CH₂-O)ₙ] n indicating the number of the members and being defined as any integer, and 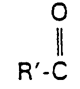 is an amino acid radical, a peptide radical or a glycopeptide radical,
if necessary deprotecting the N-terminal amino group of the compounds of the formula I and linking the compound of the formula I with N-protected amino acids to give higher peptide units, whereupon the protective group at the carboxyl end is eliminated by means of a lipase dissolved in water or aqueous solutions, with enzymatic catalysis.

2. The process as claimed in claim 1, wherein the hydrocarbon bridges between the polar members in the radical R are ethylene members of the structure -CH₂-CH₂-.

3. The process as claimed in claim 1 or 2, wherein the polar members between the ethylene bridges in the radical R are oxygen.

4. The process as claimed in claim 1 or 2, wherein the polar members between the ethylene bridges in the radical R are nitrogen functions of the structure N-R where R = H or R = lower alkyl (C₁-C₆), it also being possible for these to be linked with the ethylene bridges to form rings.

5. The process as claimed in any of claims 1 to 4, wherein the polar members in the radical R are both oxygen and amine nitrogen functions.

6. The process as claimed in any of claims 1 to 5, wherein the group R'-CO is a peptide radical composed of 1 to 100 amino acids.

7. The process as claimed in claim 6, wherein the peptide radical is composed of 1 to 50 amino acids.

8. The process as claimed in claim 7, wherein the peptide radical is composed of 1 to 25 amino acids.

9. The process as claimed in any of claims 1 to 8, wherein the group R'-CO is a glycopeptide radical.

10. The process as claimed in any of claims 1 to 9, wherein R in formula I is a diethylene glycol monomethyl ether radical (DEM).

11. The process as claimed in claim 10, wherein a compound of the formula R'-COOH in which R' has the meanings given in claim 1 is reacted with an alcohol of the formula R-OH in which R has the meaning given in claim 10 under the conditions of azeotropic esterification to give a compound of the formula I as claimed in claim 10.

12. The process as claimed in any of claims 1 to 9, wherein the compound of the formula I is a 2-(morpholino)ethyl ester.

13. The process as claimed in any of claims 1 to 9, wherein the compound of the formula I is a polyethylene glycol (PEG) ester.

14. The process as claimed in claim 12, wherein a haloalkyl ester of the formula R'-COO-CH₂-CH₂-X in which X is Cl, Br or I is reacted with morpholine to give the 2-(morpholino)ethyl ester.

15. The process as claimed in any of claims 1 to 14, wherein the enzymatic hydrolysis is effected by catalysis with a lipase dissolved in water or aqueous solutions in the presence of complexing ions, preferably alkali metal ions.

## Revendications

1. Procédé de synthèse peptidique, caractérisé en ce que
l'on estérifie un amino acide, un peptide ou un glycopeptide, dont le groupe amino N-terminal est protégé ou non protégé et qui peut porter d'autres groupes fonctionnels sous forme protégée ou non protégée, en un composé de formule générale I protégé sur la fonction carboxy terminale 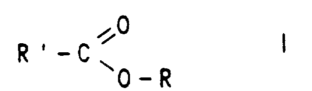 formule dans laquelle,
R représente un radical organique ramifié ou non, qui contient en tant que chaînons polaires entre des ponts hydrocarbonés aliphatiques ou araliphatiques des atomes d'oxygène de fonction éther, des groupements azotés de type amine, ou un mélange de groupements éther et amine, qui peuvent être liés en une structure cyclique, la longueur totale n'excédant pas 20 chaînons, dans le cas de polyéthylèneglycol [(CH₂-CH₂-O)ₙ], n représente le nombre de chaînons et est défini comme un nombre entier de grandeur quelconque et 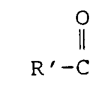
représente un résidu d'amino acide, de peptide ou de glycopeptide,
l'on déprotège si nécessaire le groupe amino N-terminal du composé de formule I et
l'on couple le composé de formule I avec des amino acides protégés sur l'azote pour former des unités peptidiques supérieures,
après quoi on élimine le groupe protecteur de la fonction carboxy terminale par catalyse enzymatique au moyen d'une lipase dissoute dans de l'eau ou dans des solutions contenant de l'eau.

2. Procédé selon la revendication 1, caractérisé en ce que les ponts hydrocarbonés entre les chaînons polaires dans le radical R sont des chaînons éthylène de structure -CH₂-CH₂-.

3. Procédé selon la revendication 1 ou 2, caractérisé en ce que les chaînons polaires entre les ponts éthylène dans le radical R sont des atomes d'oxygène.

4. Procédé selon la revendication 1 ou 2, caractérisé en ce que les chaînons polaires entre les ponts éthylène dans le radical R sont des fonctions azotées de structure N-R où R = H ou R = alkyle inférieur en (C₁-C₆), qui peuvent être liées avec les ponts éthylène pour former des cycles.

5. Procédé selon l'une des revendications 1 à 4, caractérisé en ce que les chaînons polaires dans le radical R sont aussi bien des atomes d'oxygène que des fonctions azotées de type amine.

6. Procédé selon l'une des revendications 1 à 5, caractérisé en ce que le groupement R'-CO est un résidu peptidique, qui est constitué de 1 à 100 amino acides.

7. Procédé selon la revendication 6, caractérisé en ce que le résidu peptidique est constitué de 1 à 50 amino acides.

8. Procédé selon la revendication 7, caractérisé en ce que le résidu peptidique est constitué de 1 à 25 amino acides.

9. Procédé selon l'une des revendications 1 à 8, caractérisé en ce que le groupement R'-CO est un résidu de glycopeptide.

10. Procédé selon l'une des revendications 1 à 9, caractérisé en ce que R dans la formule I est un radical éther monométhylique de diéthylèneglycol (Dem).

11. Procédé selon la revendication 10, caractérisé en ce que l'on fait réagir un composé de formule R'-COOH dans laquelle R' a la signification indiquée dans la revendication 1, avec un alcool de formule R-OH, dans laquelle R a la signification indiquée dans la revendication 10, dans des conditions d'estérification azéotrope pour donner un composé de formule I selon la revendication 10.

12. Procédé selon l'une des revendications 1 à 9, caractérisé en ce que le composé de formule I est un ester 2-(morpholino)-éthylique.

13. Procédé selon l'une des revendications 1 à 9, caractérisé en ce que le composé de formule I est un ester de polyéthylèneglycol (PEG).

14. Procédé selon la revendication 12, caractérisé en ce que l'on fait réagir un ester d'alkyle halogéné de formule R'-COO-CH₂-CH₂-X, dans laquelle X représente Cl, Br ou I, avec de la morpholine pour former l'ester 2-(morpholino)-éthylique.

15. Procédé selon l'une des revendications 1 à 14, caractérisé en ce que l'hydrolyse enzymatique est réalisée par catalyse avec une lipase dissoute dans de l'eau ou dans des solutions contenant de l'eau en présence d'ions complexants, de préférence d'ions alcalins.
